# EUROPÄISCHE PATENTANMELDUNG

(11) **EP 0 916 352 A2**
(43) Veröffentlichungstag der Anmeldung: **19.05.1999**
(21) Anmeldenummer: 98108565.7
(22) Anmeldetag: 12.05.1998
(51) Int. Cl.: A61L 33/00, A61L 31/00, A61L 27/00, A61L 29/00

(54) **Verwendung von Polymeren zur Inhibierung der Denaturierung von adsorbierten Eiweissstoffen**

(30) Priorität: 31.05.1997 DE 19722952
(71) Anmelder: HÜLS AKTIENGESELLSCHAFT, 45764 Marl (DE)
(72) Erfinder: Lohr, Frauke, Dr., 45657 Recklinghausen (DE); Pawlik, Andreas, Dr., 38159 Vechelde (DE); Bree, Martina, Dr., 10709 Berlin (DE); Motschmann, Hubert, Dr., 12055 Berlin (DE); Vieira, Euridice, 4810 Guimaraes (PT)

(57) **Zusammenfassung**

Die Erfindung betrifft die Verwendung von Polymeren, die zumindest an der Oberfläche Struktureinheiten mit 1, 2 oder 3 Fluoratomen an einem ersten Kohlenstoffatom aufweisen, das an ein zweites Kohlenstoffatom gebunden ist, welches eine Hydroxylgruppe trägt und weiterhin durch eine weitere Trifluormethylgruppe substituiert ist, sofern das erste Kohlenstoffatom 3 Fluoratome trägt, als die Denaturierung von immobilisierten Eiweißstoffen inhibierendes Material. Die Erfindung betrifft weiter Erzeugnisse, die aus einem solchen bestehen oder Teile aus einem solchen Polymeren enthalten und bei deren bestimmungsgemäßer Verwendung diese Polymeren mit nativen Eiweißstoffen in Berührung kommen, deren Denaturierung verhindert werden soll.

## Beschreibung

Die Erfindung betrifft die Verwendung von bestimmten Polymeren zur Inhibierung der Denaturierung von adsorbierten Eiweißstoffen. Die Erfindung betrifft weiterhin Erzeugnisse, wie Geräte, Instrumente und andere Gegenstände, die aus den Polymeren bestehen oder Teile aus diesen Polymeren enthalten, und betrifft die Verwendung dieser Erzeugnisse für medizinische und biotechnische Zwecke.

In der Medizintechnik werden häufig Geräte oder andere Gegenstände aus polymeren Materialien verwendet, die kurz-, mittel- oder längerfristig mit Körperflüssigkeiten, wie Lymphe und Blut, oder mit Gewebe in Berührung kommen. Solche Geräte und Gegenstände sind z.B. Implantate, Sonden, Katheter, Drainageschläuche, Stents, Herzklappen und Herzschrittmacher. An deren Oberflächen werden im Körper befindliche Eiweißstoffe, z.B. Immunoglobuline und Gerinnungsfaktoren, wie Thrombin, oder Eiweißstoffe enthaltende Zellen, die in Körperflüssigkeiten oder im Gewebe vorkommen, wie Makrophagen, Blutzellen, Gewebezellen und Bakterien, adsorbiert. Die Adsorption solcher biologischen Spezies ist in der Regel mit einer Konformationsänderung verbunden, die zu einem Verlust der biologischen Wirksamkeit führt. Derartige strukturelle Änderungen werden im allgemeinen als Denaturierung bezeichnet. Dabei bricht die intramolekulare Wechselwirkung, vornehmlich über Wasserstoffbrücken, und damit auch die α-Helix-Struktur zusammen, die nahezu allen Eiweißstoffen zumindest in Teilbereichen zueigen ist. Denaturierte Eiweißstoffe begünstigen die Besiedelung der Oberflächen mit Bakterien, die wiederum zu entzündlichen Prozessen Anlaß geben, wie sie nicht selten bei Patienten eintreten, die unter Verwendung solcher Geräte oder Gegenstände behandelt werden. Adhärierende und dadurch aktivierte Thrombozyten können in einer vielstufigen Reaktionskaskade zur Bildung von Thromben bzw. zu einer Embolie führen. Es wäre daher erwünscht, über polymere Materialien verfügen zu können, die die Denaturierung von Eiweißstoffen hemmen oder gar aufheben und somit verhindern, daß die erwähnten oder andere unerwünschte und oft verhängnisvolle Folgereaktionen ablaufen.

Die Adsorption von Eiweißkörpern spielt auch bei biotechnischen Verfahren eine Rolle, z.B. bei Bioreaktoren, bei denen Enzyme auf unterschiedlichen Trägermaterialien immobilisiert werden. Dies kann z.B. durch Quervernetzung, Bindung an den Träger durch Adsorption, ionische Wechselwirkungen oder aber durch Einschluß der Eiweißstoffe realisiert werden. Die biologische Aktivität der immobilisierten Eiweißstoffe sollte über Monate erhalten bleiben, was den Erhalt der nativen Konformation des aktiven Zentrums erfordert.

Daneben ist die Nativität adsorbierter Proteine für die Affinitätschromatographie von hoher Bedeutung, die der Isolierung oder Anreicherung von speziellen Eiweißstoffen aus komplexen biologischen Systemen, wie Blut oder Lymphe, dient. Bei der Verwendung von polymeren Werkstoffen, z.B. als Säulenmaterialien, für Lagerbehälter oder Schläuche sowie als Membranen zur Fixierung von Enzymen, besteht die Gefahr, daß ein Teil der Eiweißstoffe, sei es durch ungewollte Adsorption oder durch erwünschte Immobilisierung, dauerhaft denaturiert wird.

Es ist eine Aufgabe der Erfindung, bestimmte im Prinzip bekannte polymere Materialien für einen neuen Zweck, nämlich die Inhibierung der Denaturierung von adsorbierten Eiweißstoffen, bereitzustellen. Eine weitere Aufgabe der Erfindung besteht darin, Geräte und andere Gegenstände zur Verfügung zu stellen, die diese polymeren Materialien enthalten und für Zwecke verwendbar sind, bei denen die Denaturierung von adsorbierten Eiweißstoffen inhibiert werden soll.

Einer der Gegenstände der Erfindung ist die Verwendung von Polymeren, die zumindest an der Oberfläche Struktureinheiten mit 1, 2 oder 3 Fluoratomen an einem ersten Kohlenstoffatom aufweisen, das an ein zweites Kohlenstoffatom gebunden ist, welches eine Hydroxylgruppe trägt und weiterhin durch eine weitere Trifluormethylgruppe substituiert ist, sofern das erste Kohlenstoffatom 3 Fluoratome trägt, als die Denaturierung von immobilisierten Eiweißstoffen inhibierendes Material.

Weiterhin sind ein Gegenstand der Erfindung Geräte und Gegenstände, die aus den genannten Polymeren bestehen oder Teile aus den genannten Polymeren enthalten und bei deren bestimmungsgemäßer Verwendung diese Polymeren mit nativen Eiweißstoffen in Berührung kommen, deren Denaturierung verhindert werden soll.

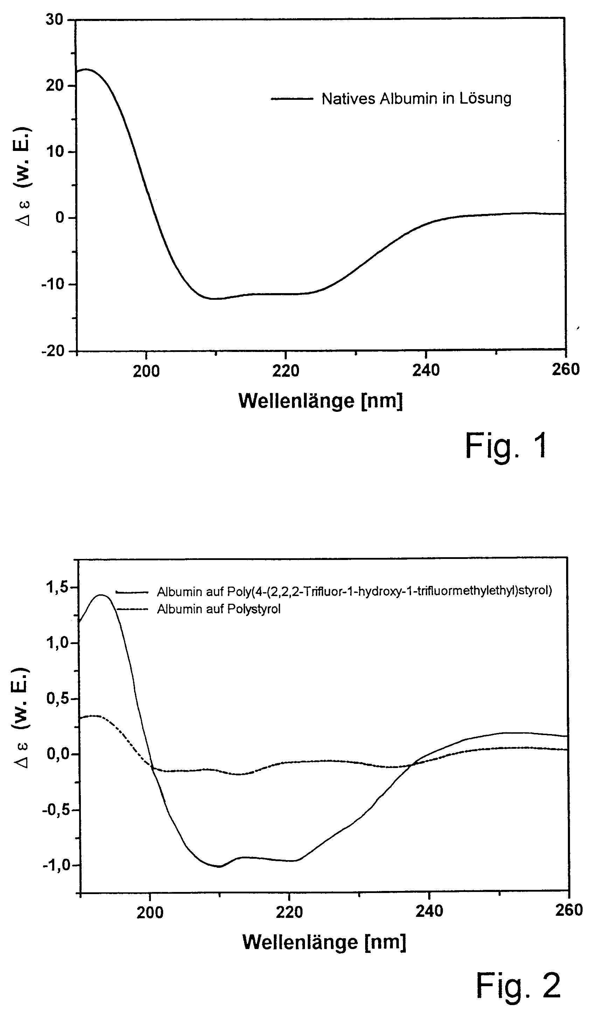

Aus den Spektren der Figuren 1 bis 4 ist ersichtlich, daß Humanserumalbumin (HSA) und Humanfibrinogen (HFb) bei der Adsorption an Polymeren mit den erwähnten Struktureinheiten ihre native Form beibehalten, während bei der Adsorption an Polystyrol Denaturierung eintritt.

Überraschenderweise wird die Denaturierung von Eiweißstoffen, die als solche oder als Bestandteile von Zellen in Körperflüssigkeiten oder im Gewebe enthalten sind, durch die erfindungsgemäße Verwendung von Polymeren mit mindestens einem Fluoratom und einer Hydroxylgruppe an vicinalen Kohlenstoffatomen wirkungsvoll und nachhaltig inhibiert. Diese Wirkung wird verstärkt, wenn mehr als nur ein Fluoratom an dem Kohlenstoffatom steht, das dem Kohlenstoffatom mit der Hydroxylgruppe benachbart ist. Dies ist z.B. bei Polymeren der Fall, die die Gruppe -C(OH)(CF₃)₂ enthalten. Es kann offen bleiben, ob die erfindungsgemäßen Polymeren die Denaturierung nativer Eiweißstoffe verhindern oder denaturierte Eiweißstoffe unverzüglich renaturieren. Im Rahmen dieser Erfindung soll die Bezeichnung "inhibieren" beide Möglichkeiten einschließen.

Polymere mit den erwähnten Struktureinheiten, die für die vorliegende Erfindung verwendet werden, sind bekannt. So werden Polymere, die ein einfach fluorsubstituiertes Kohlenstoffatom enthalten, das an ein Kohlenstoffatom mit Hydroxylgruppe gebunden ist, in SU-802429 beschrieben. Ferner sind eine Reihe von Polymeren mit Struktureinheiten bekannt, die eine Trifluormethylgruppe enthalten, welche an ein Kohlenstoffatom mit Hydroxylgruppe gebunden ist, das eine weitere Trifluormethylgruppe trägt (DE-A-1957963, DE-A-2021069, DE-A-4207261, DE-A-4207264, US-A 3414549, US-A 3438946, US-A 3444148, US-A-3549705, US-A 3696081, US-A-4640965, US-A-4690993, US-A-5241007, US-A-5320716, US-A-5506309, US-A-5532106, US-A-5536616, JA-A-01293337, JA-A-02120354, JA-A-02120355, JA-A-02120356, JA-A-05303907, JA-A-07120926, JA-A-50143888, JA-A-52023192, JA-A-52076299, JA-A-55102629, JA-A-57194276, JA-A-61284761, JA-A-62014647, JA-A-62015543, JA-A-62143046, JA-A-62186907, JA-A-62240953, FR-A-1541003, FR-A-2074835, EP-A-0043948, EP-A-0057065, WO-A-8809799, WO-A-9319111). Die bekannten Polymeren wurden für die verschiedensten Verwendungen vorgeschlagen, z.B. für Anstrichmittel mit Korrosionsschutz, Beschichtungsmittel für Halbleitervorrichtungen, Finishmittel, Filter, Membranen (z.B. für Elektrolysezellen), elektrochrome Vorrichtungen, Kondensatoren und hydraulische Flüssigketen. Die in den erwähnten US-A-4640965 und US-A-4690933 beschriebenen Polymeren sollen besonders gasdurchlässig sein und sich für Kontaktlinsen und Intraokularlinsen eignen. In keiner der Vorveröffentlichungen ist jedoch erwähnt, daß solche Polymere die Denaturierung von immobilisierten Eiweißstoffen inhibieren.

Gruppen mit der erfindungswesentlichen Struktureinheit sind die folgenden: 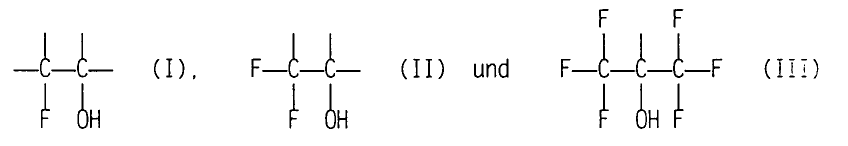 Die erfindungsgemäß zu verwendenden Polymeren haben ein Polymergerüst, das durch beliebige bekannte Aufbaureaktionen entstanden sein kann. Dazu gehören durch radikalische Polymerisation von Monomeren mit olefinischer Doppelbindung entstandene Polymergerüste ebenso wie Polymergerüste, die auf andere Weise synthetisiert wurden, z.B. durch Polykondensationsreaktionen, wie Polyamide und gesättigte Polyester; durch Polyadditionsreaktionen, wie Polyurethane, Polyharnstoffe oder Polyalkylenglykole; durch Äquilibrierungsreaktionen, wie Polysiloxane; oder durch eine Kombination der genannten Reaktionstypen.

Die erwähnten charakteristischen, wirkungsbestimmenden Struktureinheiten können schon während der Synthese der Polymeren durch Verwendung entsprechender Monomerer eingeführt werden. Sie befinden sich dann sowohl auf der Oberfläche als auch im Bulk. Alternativ können diese Struktureinheiten auch durch nachträgliche Modifizierung von Polymeren geschaffen werden. In diesem Falle finden sich die charakteristischen Struktureinheiten, je nach der Methode der Modifizierung, auf der Oberfläche und im Bulk oder nur auf der Oberfläche des Polymers.

Für die Herstellung der erfindungsgemäß zu verwendenden Polymeren geeignete Monomere sind u.a.: p-(2-Hydroxyhexafluorisopropyl)-styrol, 1,1,1-Trifluor-6,6-dimethyl-4-methylen-2-(trifluormethyl)-2-heptanol, 1,1,1-Trifluor-4-phenyl-2-(trifluormethyl)-4-penten-2-ol, 5,5,5-Trifluor-4-hydroxy-2-methylen-4-(trifluormethyl)-valeriansäuremethylester, N-[2,2,2-Trifluor-1-hydroxy-1-(trifluormethylethyl)]-2-propenamid, 1,1,1-Trifluor-2-(trifluormethyl)-4-penten-2-ol, 1,1,1,3,3,3-Hexafluor-2-(2-propenyloxy)-2-propanol, 1,1,1,3,4,4-Hexafluor-2-(trifluormethyl)-3-buten-2-ol, 1,1,1-Trifluor-2-(trifluormethyl)-3-buten-2-ol, 1,1,1,3,3,4,4-Heptafluor-4-[(trifluorethenyl)oxy]-2-(trifluormethyl)-2-butanol, 1,1,1,2,3,3,4,4-Octafluor-4-[(trifluorethenyl)oxy]-2-butanol. β-[Bis(trifluormethyl)hydroxymethyl]-styrol sowie die Monomeren der Formeln 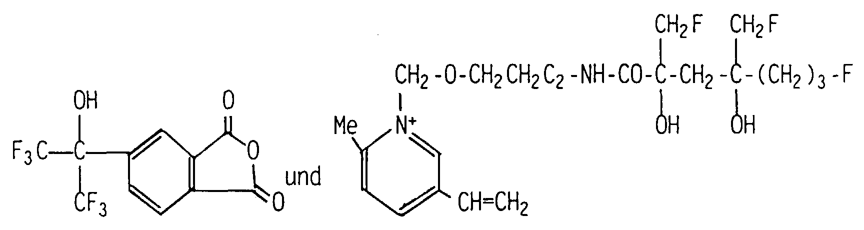 Diese und andere Monomere sind überwiegend aus den erwähnten Patentschriften oder aus Veröffentlichungen bekannt, in denen auch die Herstellung von (Co)polymeren mit den erwähnten charakteristischen Struktureinheiten beschrieben ist.

Da diese Monomeren jedenfalls teilweise nicht ohne weiteres zugänglich sind, setzt man sie zweckmäßig neben einem oder mehreren anderen, gut zugänglichen Monomeren ein. Geeignete Comonomere sind einfach oder zweifach olefinisch ungesättigte Monomere, wie (Meth)acrylate, z.B. Methyl-, Ethyl-, Butyl- und 2-Ethylhexyl(acrylat), (Meth)acrylnitril und (Meth)acrylamid; Vinylaromaten, z.B. Styrol, α-Methylstyrol, Vinyltoluol oder Divinylbenzol; Vinylester, z.B. Vinylacetat und Vinylpropionat; Vinylether, z.B. Vinylethylether oder Vinylbutylether; sowie andere Vinylverbindungen, z.B. Vinylchlorid, Vinylethylketon, 1- oder 2-Buten und 1-Octen.

Dabei kann das Mengenverhältnis der Monomeren in weiten Grenzen schwanken. So können die Monomeren mit den charakteristischen Struktureinheiten, je nach Monomer, in Mengen von 1 bis 100 Mol%, zweckmäßig von 2 bis 50 Mol-% eingesetzt werden. Nicht selten wird die erwünschte inhibierende Eigenschaft des Copolymers mit 2 bis 20 Mol-% des charakteristischen Monomers voll erreicht.

Vorteilhaft für manche Zwecke ist die Mitverwendung von Monomeren, die allein oder in bestimmten Kombinationen weitere erwünschte Eigenschaften erteilen oder verstärken. Solche Monomere sind z.B. Carboxylatgruppen oder Sulfonatgruppen enthaltende Monomere, die die Blutverträglichkeit und die bakterienabweisenden Eigenschaften der Polymeren verbessern. Solche Monomere sind u.a. Natrium(meth)acrylat, Natriummaleinat und Natriumfumarat sowie Natriumvinylsulfonat und Natrium-4-vinylbenzolsulfonat. Wenn diese wasserlöslichen Monomeren aufgrund der jeweiligen Polymerisationsbedingungen nicht oder nicht gut einsetzbar sind, kann man auch Monomere verwenden, deren funktionelle Gruppen nach der Polymerisation in Carboxylatgruppen bzw. Sulfonatgruppen umgewandelt werden. Solche funktionellen Gruppen sind vorzugsweise Carbon- oder Sulfonsäure-, Ester-, Anhydrid- oder Amidgruppen. Die Carboxylatgruppen bzw. Sulfonatgruppen können im Polymer beispielsweise im Molverhältnis von 1:20 bis 20:1 vorhanden sein. Statt diese Gruppen über die Mitverwendung von bestimmten Monomeren bei der Polymerisation einzubringen, kann man die Monomeren auch in an sich bekannter Weise radikalisch initiiert auf die Oberfläche pfropfen.

Die Inhibierung der Denaturierung der adsorbierten oder immobilisierten Eiweißstoffe läßt sich durch Circulardichroismus(CD)-Spektroskopie nachweisen, mit deren Hilfe die absolute Konformation von chiralen Verbindungen aufgeklärt werden kann und Konformationsänderungen verfolgt werden können. Hierbei wird linear polarisiertes Licht, im allgemeinen im sichtbaren oder im UV-Bereich, durch die z.B. in Lösung vorliegende oder auf einem durchsichtigen Medium adsorbierte chirale Verbindung gestrahlt. Liegt die eingestrahlte Wellenlänge im Absorptionsbereich der chiralen Verbindung, so können elektronische Übergänge eintreten, die zu einer elliptischen Polarisierung des zuvor linear polarisierten Lichtes führen, das als Überlagerung einer rechts circular polarisierten und einer links circular polarisierten Teilwelle mit gleichen Amplituden aufgefaßt werden kann. Die Absorptionskoeffizienten einer chiralen Verbindung für die rechts circular bzw. links circular polarisierten Teilwellen sind nicht notwendigerweise identisch. Die Differenz der molaren dekadischen Absorptionskoeffizienten für die links circular bzw. die rechts circular polarisierte Teilwelle wird als Circulardichroismus bezeichnet. Nach dem Durchgang durch die Probe überlagern sich die nach der Absorption verbliebenen Anteile der ursprünglichen Teilwellen und ergeben ein elliptisch polarisiertes Licht, da sie im allgemeinen nicht mehr die gleiche Amplitude besitzen. Ändert man die Wellenlänge des eingestrahlten Lichtes, so erhält man ein Spektrum, das den Circulardichroismus als Funktion der Wellenlänge zeigt.

Ebenso kann die Probe nacheinander mit rechts bzw. links polarisiertem Licht durchstrahlt werden, wodurch man, wie zuvor beschrieben, ein Differenzspektrum erhält, das Rückschlüsse auf die Struktur der chiralen Verbindung zuläßt, z.B. auf deren absolute Konformation.

Zu den mittels CD-Spektroskopie untersuchten chiralen Verbindungen gehören verschiedene Polypeptide und Eiweißstoffe (oder Proteine). Auch Konformationsveränderungen von Eiweißstoffen an Grenzflächen sind bereits CD-spektroskopisch untersucht worden (siehe z.B. Y.-H.Chen et al., Biochemistry, Vol.11, No. 22 (1972), 4120-4131; C.R.MacMillin et al., Journal of Colloid and Interface Science, 48, No.2, (1974) 345-349; L.J.Smith et al., Biochimica et Biophysica Acta, 1121 (1992) 111-118; W.Norde et al., Journal of Colloid and Interface Science, 112, No.2, 447-456; W.Norde et al., Colloids and Surfaces 64 (1992), 87-93).

Wenn die Substrate mit den adsorbierten Eiweißkörpern durchlässig für Licht der betreffenden Wellenlänge sind, kann man Transmissionsmessungen durchführen. Dies ist jedoch nicht möglich bei lichtundurchlässigen Substraten, für die sich Reflexionsmessungen anbieten. Voraussetzung hierfür ist jedoch, daß eine hinreichend stark reflektierende Oberfläche vorhanden ist, was häufig nicht zutrifft. Ein elegantes Verfahren zur Gewinnung von CD-Spektren adsorbierter Eiweißstoffen (und anderer chiraler Verbindungen) durch Reflexionsmessung ist in der deutschen Patentanmeldung 197 17 431.0 beschrieben.

Die erfindungsgemäßen Polymeren, die die charakteristische Struktureinheit im Bulk enthalten, lassen sich nach Verfahren zu Formteilen verarbeiten, wie sie in der Kunststofftechnik üblich sind, z.B. durch Spritzgießen, Preßspritzen, Extrudieren, Tiefziehen oder Gießen. Weiterhin kann man die erfindungsgemäßen Polymeren zur Beschichtung von Formteilen aus anderen Polymeren benutzen. Dies ist z.B. dann empfehlenswert, wenn die mechanischen Eigenschaften der erfindungsgemäß zu verwendenden Polymeren, wie Biege- oder Reißfestigkeit, den Anforderungen für bestimmte Anwendungen nicht oder nicht voll entsprechen. Besonders gute Haftung wird erzielt, wenn man das erfindungsgemäße Polymer strahleninduziert auf die aktivierte Substratoberfläche pfropft, wie dies in den deutschen Patentanmeldungen 197 000 81.9, 197 000 82.7 und 197 000 83.5 beschrieben ist.

Man kann unter Einsatz der erfindungsgemäß zu verwendenden Polymeren Erzeugnisse, wie Geräte, Instrumente und andere Gegenstände, herstellen, die aus den Polymeren bestehen oder Teile aus den Polymeren (z.B. Beschichtungen) enthalten und bei deren bestimmungsgemäßer Verwendung diese Polymeren mit nativen Eiweißstoffen in Berührung kommen, deren Denaturierung verhindert werden soll. Letzteres ist vor allem bei medizinischen und biotechnischen Anwendungen der Fall. Die Polymeren eignen sich besonders für Erzeugnisse, die mit Blut, Lymphe oder ähnlichen Körperflüssigkeiten in Berührung kommen oder biotechnischen Zwecken dienen. Solche Erzeugnisse sind u.a. Implantate, Sonden, Katheter, Drainageschläuche, Stents, Herzklappen und Herzschrittmacher sowie Behälter und Rohrleitungen.

Die folgenden Beispiele sollen die Erfindung weiter erläutern, nicht aber ihren Anwendungsbereich begrenzen, wie er in den Patentansprüchen dargelegt ist.

### Beispiel 1

Poly-[4-(2,2,2-Trifluor-1-hydroxy-1-trifluormethylethyl]-styrol wurde hergestellt, wie in JA-A-62240953 beschrieben. Ein Träger aus Quarzglas wurde durch Spin-Coaten mit eine 10%-igen Lösung des Homopolymers in THF beschichtet. Der beschichtete Träger wurde 20 min in eine 10⁻⁶-molare Lösung von Humanserumalbumin (HSA; Fa. SIGMA) in phosphatgepufferter Lösung (PBS-Puffer der Fa. SIGMA) mit einem pH-Wert von 7,4 getaucht. Anschließend wurde die Proteinlösung gegen reine Pufferlösung ausgetauscht und in Transmission ein CD-Spektrum des adsorbierten Humanserumalbumins genommen (Figur 2). Zum Vergleich wurde ein weiterer Quarzglasträger mittels Spin-Coaten einer 10%-igen Lösung von Polystyrol (VESTYRON^{(R)}116 der Fa. Hüls AG) in THF (Fa. Aldrich) mit dem Polymer beschichtet. Hierauf wurde, wie zuvor beschrieben, HSA adsorbiert und ein CD-Spektrum wiederum in Transmission gemessen (Figur 2). Des weiteren wurde für einen Vergleich mit der nativen Struktur des HSA ein CD-Spektrum einer 10⁻⁶ M Lösung von HSA in phosphatgepufferter Lösung genommen (Figur 1). Ein Vergleich der 3 Spektren zeigt, daß HSA bei der Adsorption auf dem Polymer mit den inhibierenden Struktureinheiten im Gegensatz zur Polystyroloberfläche seine native Konfiguration bei behält.

### Beispiel 2

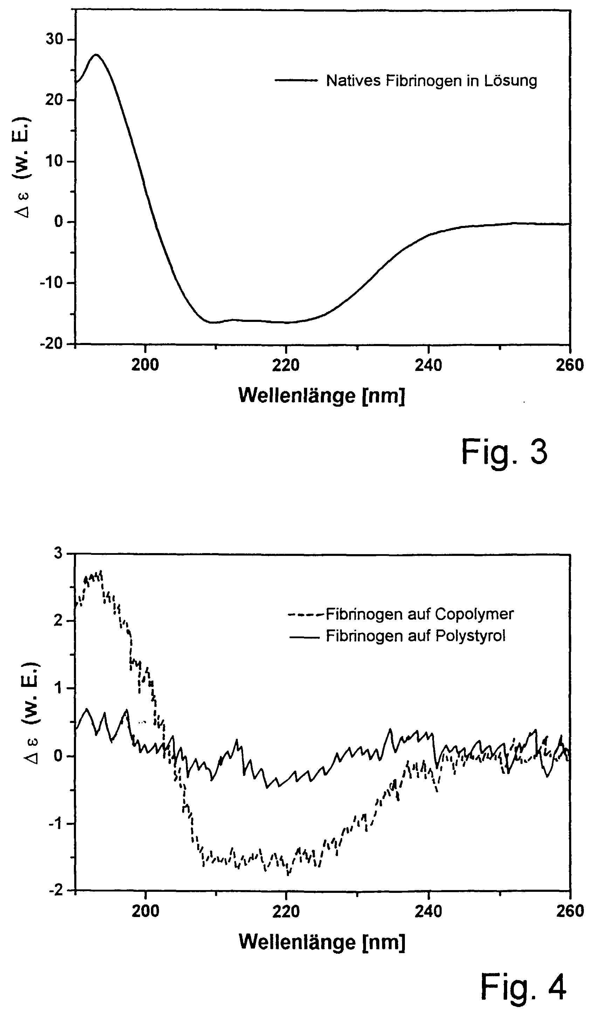

Ein Copolymer aus 4-(2,2,2-Trifluor-1-hydroxy-1-trifluormethylethyl)-styrol und Styrol (Molverhältnis 1:1) wurde synthetisiert wie in JA-A-07120926 beschrieben. Ein Träger aus Quarzglas wurde durch Spin-Coaten mit eine 10%-igen Lösung des Copolymers in THF beschichtet. Der beschichtete Träger wurde 20 min in eine 10⁻⁶-molare Lösung von Humanfibrinogen (HFb; Fa. SIGMA) in phosphatgepufferter Lösung (PBS-Puffer der Fa. SIGMA) mit einem pH-Wert von 7,4 getaucht. Anschließend wurde die Proteinlösung gegen reine Pufferlösung ausgetauscht und in Transmission ein CD-Spektrum des adsorbierten Humanfibrinogens genommen (Figur 4). Zum Vergleich wurde ein weiterer Quarzglasträger mittels Spin-Coaten einer 10%-igen Lösung von Polystyrol (VESTYRON^{(R)}116 der Fa. Hüls AG) in THF (Fa. Aldrich) mit dem Polymer beschichtet. Hierauf wurde, wie zuvor beschrieben, HFb adsorbiert und ein CD-Spektrum wiederum in Transmission gemessen (Figur 4). Des weiteren wurde für einen Vergleich mit der nativen Struktur des HFb ein CD-Spektrum einer 10⁻⁶ M Lösung von HFb in phosphatgepufferter Lösung genommen (Figur 3). Ein Vergleich der 3 Spektren zeigt, daß HFb bei der Adsorption auf dem Polymer mit den inhibierenden Struktureinheiten im Gegensatz zur Polystyroloberfläche seine native Konfiguration beibehält.

## Patentansprüche

1. Verwendung von Polymeren, die zumindest an der Oberfläche Struktureinheiten mit 1, 2 oder 3 Fluoratomen an einem ersten Kohlenstoffatom aufweisen, das an ein zweites Kohlenstoffatom gebunden ist, welches eine Hydroxylgruppe trägt und weiterhin durch eine weitere Trifluormethylgruppe substituiert ist, sofern das erste Kohlenstoffatom 3 Fluoratome trägt, als die Denaturierung von immobilisierten Eiweißstoffen inhibierendes Material.

2. Verwendung nach Anspruch 1, dadurch gekennzeichnet, daß die verwendeten Polymeren Struktureinheiten der Formeln 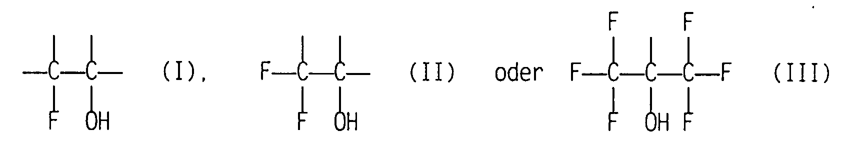 enthalten.

3. Verwendung nach Anspruch 1 oder 2, dadurch gekennzeichnet, daß die Struktureinheiten schon während der Synthese der Polymeren durch Verwendung entsprechender Monomerer eingeführt werden und sich sowohl auf der Oberfläche als auch im Bulk befinden.

4. Verwendung nach Anspruch 1 oder 2, dadurch gekennzeichnet, daß die Struktureinheiten durch nachträgliche Modifizierung von Polymeren geschaffen werden und sich auf der Oberfläche und im Bulk oder nur auf der Oberfläche des Polymers befinden.

5. Verwendung nach einem der Ansprüche 1 bis 4, dadurch gekennzeichnet, daß bei der Herstellung der Polymeren neben den Monomeren, die die genannten Struktureinheiten einführen, andere olefinisch ungesättigte Monomere mitverwendet werden.

6. Verwendung nach einem der Ansprüche 1 bis 3, dadurch gekennzeichnet, daß bei der Herstellung der Polymeren neben Monomeren, die die genannten Struktureinheiten einführen, Carboxylatgruppen oder Sulfonatgruppen enthaltende Monomere mitverwendet werden.

7. Verwendung nach einem der Ansprüche 1 bis 3, dadurch gekennzeichnet, daß nach der Herstellung des Polymers Carboxylatgruppen oder Sulfonatgruppen enthaltende Monomere auf das Polymer gepfropft werden.

8. Erzeugnisse, die aus einem der in einem der Ansprüche 1 bis 7 genannten Polymeren bestehen oder Teile aus einem solchen Polymeren enthalten und bei deren bestimmungsgemäßer Verwendung diese Polymeren mit nativen Eiweißstoffen in Berührung kommen, deren Denaturierung verhindert werden soll.

9. Erzeugnisse nach Anspruch 8, dadurch gekennzeichnet, daß sie zu medizinischer oder biotechnischer Verwendung bestimmt sind.

10. Erzeugnisse nach Anspruch 8, dadurch gekennzeichnet, daß sie zu medizinischer Verwendung, bei der sie mit Blut oder Lymphe in Berührung kommen, oder zu biotechnischer Verwendung bestimmt sind.
